# EUROPÄISCHE PATENTANMELDUNG

(11) **EP 1 174 159 A2**
(43) Veröffentlichungstag der Anmeldung: **23.01.2002**
(21) Anmeldenummer: 01117286.3
(22) Anmeldetag: 17.07.2001
(51) Int. Cl.: A61M 16/00, F04D 29/28

(54) **Vorrichtung zur Zufuhr eines Atemgases**

(30) Priorität: 19.07.2000 DE 20012654 U
(71) Anmelder: MAP Medizintechnik für Arzt und Patient GmbH & Co. KG, 82152 Martinsried (DE)
(72) Erfinder: Burz, Johann Sebastian, 86191 Utting (DE); Mayer, Wolfgang, 79285 Ebringen (DE); Heidmann, Dieter, 82541 Münsing (DE); Vögele, Harald, 82131 Gauting (DE); Madaus, Stefan, 82152 Krailling (DE)
(74) Vertreter: Rössig, Rolf

(57) **Zusammenfassung**

Die Erfindung betrifft eine Vorrichtung zur Zufuhr eines Atemgases zu einem Patienten. Derartige Vorrichtungen finden insbesondere Anwendung im Bereich der Schlaftherapie. Erfindungsgemäß wird vorgeschlagen, eine in diesem Zusammenhang verwendete Gebläseeinrichtung mit einem Laufrad zu versehen, wobei das Laufrad mehrere durch Schaufeln voneinander getrennte Schaufelkanäle aufweist. Das erfindungsgemäße Laufrad zeichnet sich dadurch aus, dass die Anzahl der Schaufeln derart festgelegt ist, dass sich am Außenumfang des Rades eine Schaufelkanalbreite im Bereich von 0,1 bis 6,5% des Außenumfangs des Laufrades ergibt. Dadurch wird es auf vorteilhafte Weise möglich, ein Laufrad zu schaffen das sich durch eine hohe Laufruhe auszeichnet und der Atemgasdruck auf vorteilhafte Weise an die momentanen physiologischen Bedürfnisse eines Patienten verbessert anpassbar ist.

## Beschreibung

Die Erfindung betrifft eine Vorrichtung zur Zufuhr eines Atemgases zu einem Patienten. Derartige Vorrichtungen finden insbesondere Anwendung im Bereich der Schlaftherapie. Durch die Zufuhr eines Atemgases unter Überdruck, beispielsweise im Bereich von 4 bis 18 mbar, wird es auf physiologisch gut verträgliche Weise möglich, eine pneumatische Schienung der oberen Atemwege eines Patienten zu erreichen. Hierdurch kann auf wirkungsvolle Weise etwaigen Obstruktionen in diesem Atemwegsbereich vorgebeugt werden.

Bei der Durchführung einer CPAP-Therapie wird ein Atemgas, beispielsweise Umgebungsluft über eine flexible Schlauchleitung einer in abdichtender Weise auf dem Nasenbereich eines Patienten applizierten Nasenmaske, zugeführt. Im Hinblick auf einen möglichst hohen Therapiekomfort sind bei den bekannten Vorrichtungen üblicherweise Schalldämpfer vorgesehen, um die Ausbreitung von Schallereignissen über die Schlauchleitung zu dem Patienten zu verhindern. Hinsichtlich dieser Schalldämmeinrichtungen besteht jedoch das Problem, daß mit einer Steigerung des Schallabsorptionsvermögens auch der Strömungswiderstand des Atemgasleitungsweges zunimmt. Hierdurch wird die patientenseitig während eines Expirationsvorganges aufzubringende Atemarbeit erhöht.

Der Erfindung liegt die Aufgabe zugrunde, eine Vorrichtung zur Zufuhr eines Atemgases zu einem Patienten zu schaffen, die sich durch einen geringen respiratorischen Widerstand sowie eine geringe Schallemission auszeichnet.

Diese Aufgabe wird erfindungsgemäß gelöst durch eine Vorrichtung zur Zufuhr eines Atemgases mit einer Gebläseeinrichtung, die eine Antriebseinrichtung und ein Laufrad aufweist, wobei das Laufrad mehrere, durch Schaufeln voneinander getrennte Schaufelkanäle aufweist, wobei die Anzahl der Schaufeln derart festgelegt ist, daß sich am Außenumfang des Rades eine Schaufelkanalbreite im Bereich von 0,1 - 6,5 % des Außenumfangs des Laufrades ergibt.

Hierdurch wird auf überraschend vorteilhafte Weise die Intensität der unmittelbar durch das Laufrad verursachten Laufgeräusche vermindert und zudem das Frequenzspektrum der in das Atemgas eingekoppelten Schallereignisse in einen Frequenzbereich verschoben, der seitens des Patienten subjektiv als weniger unangenehm bewertet wird.

In weiterhin vorteilhafter Weise wird durch die erfindungsgemäße Ausgestaltung des Laufrades Laufrades die p/v-Charakteristik derart verändert, daß sich hinsichtlich der Anpassung der Laufraddrehzahl ein verminderter Regelbedarf ergibt.

Gem. einer besonders bevorzugten Ausführungsform der Erfindung liegt bei einem Laufrad mit einem Laufradaußendurchmesser die Anzahl der Schaufeln im Bereich von 26 - 47.

Eine im Hinblick auf die statistisch häufugsten Atmungscharakteristika vorteilhafte Ausgestaltung des Laufrades ist dadurch gegeben, daß dieses bei einem Durchmesser von 74 - 80 mm, 35 Schaufeln aufweist.

Gem. einer besonders bevorzugten Ausführungsform der Erfindung sind die Schaufeln lediglich schwach rückwärts gekrümmt ausgebildet. Hierdurch wird es möglich die p/v-Kennlinie der Fördereinrichtung derart abzuflachen, daß im Rahmen eines Expirationsvorganges der Förderdruck durch Absenken der Drehzahl in einem vergleichsweise engen Drehzahlbereich um einen Druckpegel abgesenkt werden kann der im wesentlichen dem Strömungswiderstand des Atemgasleitungssystems während des Ausatmens entspricht.

Eine im Hinblick auf einen besonders geringen respiratorischen Widerstand vorteilhafte Charakteristik der Fördereinrichtung wird gem. einem besonderen Aspekt der Erfindung dadurch erreicht, daß die in Umfangsrichtung abfolgend angeordneten Schaufelelemente unterschiedliche radiale Tiefen aufweisen. Gem. einer besonders bevorzugten Ausführungsform der Erfindung ist jedes zweite Schaufelelement verkürzt ausgebildet. Der Verkürzungsgrad liegt bezogen auf die radiale Schaufeltiefe der größeren Schaufeln vorzugsweise im Bereich von 10 - 40 %. Hierdurch ergibt sich insbesondere im Nabenbereich des Laufrades ein vergleichsweise großer freier Querschnitt.

Das Laufrad ist gem. einem weiteren Aspekt der vorliegenden Erfindung vorteilhafterweise als einseitig offenes Radiallaufrad ausgebildet. Hierdurch wird es möglich, bereits die Entstehung etwaiger Schallereignisse in den Laufradkanälen zu vermindern.

Gem. einer besonders bevorzugten Ausführungsform der Erfindung grenzt unmittelbar an die offene Seite des Laufrades eine Wandung mit schallabsorbierenden Eigenschaften an.

Diese schallabsorbierenden Eigenschaften können vorzugsweise durch eine definierte Aufrauhung der Oberfläche erreicht werden. Eine derartige Aufrauhung kann durch eine Errodierbehandlung eines entsprechenden Formwerkzeuges oder beispielsweise auch durch einen Nachbehandlungsprozeß, insbesondere Sandstrahlen, erreicht werden.

Es ist auch möglich, die angrenzende Wandung aus einem Material mit geringem Schallreflexions-, sowie vorzugsweise auch geringem Körperschall-Übertragungsvermögen auszubilden. Eine unter fertigungstechnischen Gesichtspunkten kostengünstig realisierbare Maßnahme besteht hierbei darin, daß die unmittelbar dem Laufrad zugewandte, die Laufradkanäle abdeckende stationäre Wandung durch ein elastomeres Material - vorzugsweise eine Folie aus Silikonkautschuk - gebildet ist.

Gem. einer weiteren besonders bevorzugten Ausführungsform der Erfindung weist das Laufrad insbesondere im Bereich der Laufradkanäle ebenfalls eine vergleichsweise rauhe Oberfläche auf. Hierdurch wird ebenfalls in vorteilhafter Weise das Absorptionsvermögen innerhalb der Laufradkanäle erhöht.

Die axiale Tiefe des Laufrades liegt gem. einem besonderen Aspekt der vorliegenden Erfindung vorzugsweise im Bereich von 7 bis 20 % des Laufradaußendurchmessers.

Das Laufrad weist vorzugsweise einen scheibenartigen Basiskörper auf, dessen Wandstärke zum Nabenbereich des Laufrades hin zunimmt. Die Zentrierung des Laufrades erfolgt vorzugsweise durch eine zentrale Paßbohrung, die selbstzentrierend auf einem entsprechenden Zapfenabschnitt einer Antriebseinrichtung aufsetzbar ist. Alternativ hierzu oder auch in Kombination mit dieser Maßnahme ist es möglich, den Basiskörper des Laufrades mit einem Ringabschnitt zu versehen, der ebenfalls in zentrierender Weise auf eine entsprechende Gegenstruktur einer Antriebseinrichtung beispielsweise auf einen stirnnahen Umfangsbereich eines Außenläufers eines Gebläsemotores, aufsetzbar ist.

Die Fixierung des Laufrades an der Antriebseinrichtung kann in an sich bekannter Weise durch Befestigungsmittel, wie beispielsweise Schrauben, erfolgen.

Sofern Schrauben verwendet werden, werden diese gem. einer bevorzugten Ausführungsform der Erfindung hinsichtlich ihres Gewichts sortiert. Alternativ hierzu oder auch in Kombination hiermit ist es möglich, Rastverbindungseinrichtngen vorzusehen, durch welche das Laufrad unmittelbar mit einer Antriebseinrichtung koppelbar ist.

Eine unter fertigungstechnischen Gesichtspunkten vorteilhafte Ausführungsform der Erfindung ist dadurch gegeben, daß das Laufrad ist vorzugsweise als reines Radiallaufrad ausgebildet ist. Alternativ hierzu ist es auch möglich, das Laufrad als Halb-Radiallaufrad auszubilden. Hierdurch wird eine verbesserte Selbststabilisierung des Laufrades und eine geringere Belastung der Drehlagerung des Laufrades erreicht.

Die Schaufeln weisen vorzugsweise unterschiedliche radiale Schaufeltiefen auf. Die Schaufeln sind vorzugsweise derart angeordnet, daß in Umfangsrichtung Schaufeln mit großer Schaufeltiefe und Schaufeln mit kleiner radialer Schaufeltiefe abfolgend angeordnet sind. Die radiale Schaufeltiefe der kleinen Schaufeln liegt hierbei vorzugsweise im Bereich von 30 - 70 % der radialen Schaufeltiefe der großen Schaufeln. Die axiale Schaufeltiefe (t) der Schaufelkanäle liegt vorzugsweise im Bereich von 5 - 20 % des Laufraddurchmessers (D), wobei die axiale Schaufeltiefe (t) der Schaufelkanäle entlang des Verlaufs des Schaufelkanals in radialer Richtung vorzugsweise im wesentlichen konstant ist.

Insbesondere bei der Ausgestaltung des Laufrades als Hlab-Radialrad ist es jedoch auch möglich, diese derart auszubilden, daß die Schaufeltiefe (t) der Schaufelkanäle sich im Verlauf des Schaufelkanals in radialer Richtung, ändert. Vorzugsweise ist hierbei die Ausgestaltung derart getroffen, daß die Schaufeltiefe (t) der Schaufelkanäle in radialer Richtung nach außen hin abnimmt.

Das Laufrad ist weiterhin vorzugsweise derart ausgestaltet, daß der Innendurchmesser des Zutrittsbereiches im Bereich von 25 - 66 % des Laufraddurchmessers (D) liegt.

Eine im Hinblick auf eine besonders hohe Laufruhe der Vorrichtung vorteilhafte Ausführungsform des Laufrades ist in vorteilhafter Weise dadurch gegeben, daß das Laufrad aus einem Kunststoffmaterial gebildet ist.

Die Wandungsfläche der die Schaufelkanäle begrenzenden Wandung weist vorzugsweise eine hohe Oberflächenrauhigkeit auf.

Gemäs einemweiteren Aspekt der vorliegenden Erfindung wird die eingangs angegebene Aufgabe auch gelöst durch ein Laufrad für eine Gebläseeinrichtung zum Aufbau eines Überdruckes in Atemgas, wobei das Laufrad eine Vielzahl von Laufschaufeln aufweist, wobei das Laufrad sich dadurch auszeichnet, daß die Laufschaufeln unterschiedliche radiale Schaufeltiefen aufweisen.

Vorzugsweise ist hierbei zwischen zwei Laufschaufeln großer radialer Tiefe wenigstens eine Laufschaufel verminderter radialer Tiefer angeordnet. In weiterhin vorteilhafter Weise ist hierbei ein zwischen zwei Laufschaufeln großer radialer Tiefe begrenzter Kanalbereich durch eine oder mehrere radial kürzere oder axial niedrigere Schaufelelemente unterteilt.

Die Wanddicke der Laufschaufeln nimmt vorzugsweise zum Nabenbereich hin ab. Die Wanddicke der Laufschaufeln am Außenumfang liegt vorzugsweise im Bereich von 0,6 - 1,8 mm.

Eine im Hinblick auf eine besonders stabile Drucregelung vorteilhafte Ausführungsform des Laufrades ist dadurch gegeben, daß die Laufschaufeln schwach rückwärts gekrümmt ausgebildet sind.

Weitere Einzelheiten und Merkmale ergeben sich aus der nachfolgenden Beschreibung in Verbindung mit der Zeichnung. Es zeigen:
- **Fig.1**: eine perspektivische Ansicht eines Radial-Laufrades gemäß einer ersten bevorzugten Ausführungsform der Erfindung mit 38 Schaufeln, wobei Schaufeln unterschiedlicher radialer Tiefe wechselweise angeordnet sind;
- **Fig.2a**: eine draufsicht auf das Laufrad gem. Fig.1 zur Verdeutlichung bevorzugter Krümmungsradien der hier rückwärts gekrümmten Schaufeln;
- **Fig.2b**: eine Seitenansicht des Laufrades nach Fig.2a zur Veranschaulichung der axialen Tiefe des Radiallaufrades;
- **Fig.3**: eine Skizze zur Erläuterung des Aufbaues einer Gebläseeinrichtung für ein CPAP-Gerät mit einem Radial-Laufrad unterschiedlicher radialer Schaufeltiefe und zu einem Nabenanschnitt hin abfallend auslaufenden Anströmkanten.

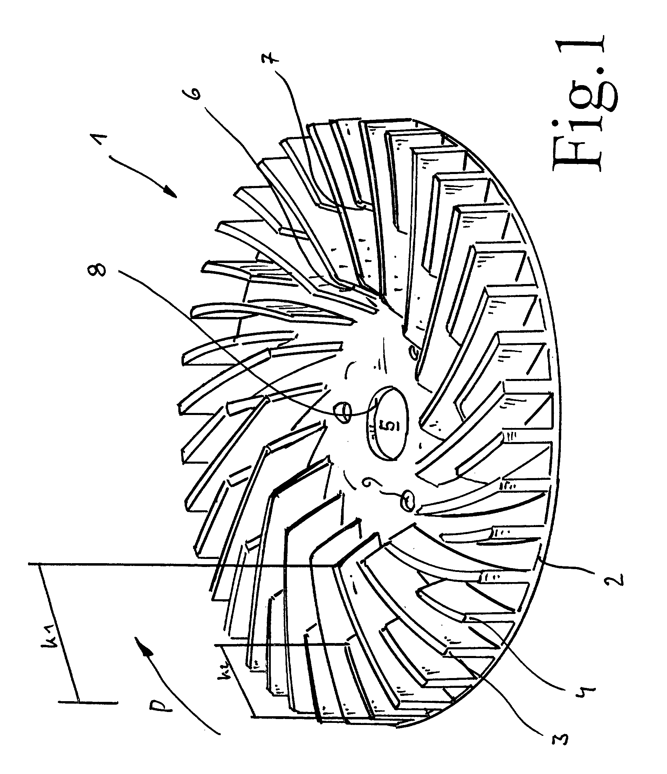

Das in Fig.1 dargestellte Laufrad 1 für ein CPAP-Gerät umfaßt einen hier scheibenartig ausgebildeten Basiskörper 2. Integral mit dem Basiskörper 2 sind hier 38 Schaufeln 3, 4 vorgesehen. Die Schaufeln 3, 4 sind bezogen auf die hier durch den Pfeil P angedeutete Drehrichtung schwach rückwärts gekrümmt ausgebildet.

Die Schaufeln 3, 4 weisen hier unterschiedliche radiale Schaufeltiefen k1, k2 auf. Bei der hier dargestellten Ausführungsform sind zwei unterschiedliche radiale Schaueltiefen vorgesehen, wobei jeweils zwischen zwei langen Schaufeln eine kurze Schaufel vorgesehen ist.

Die zum Nabenbereich 5 des Laufrades 1 weisenden Anströmkanten 6, 7 sind hier geneigt ausgebildet. Die Übergangsbereiche zwischen den einezelnen Wandungszonen der Schaufeln 3, 4 sind gerundet ausgebildet.

Im Nabenbereich 5 ist eine Zentrieröffnung 8 vorgesehen zur Aufnahme eines Zentrierabschnittes einer Antriebseinrichtung.

Zur Befestigung des Laufrades 1 an der Antriebseinrichtung sind mehrere Bohrungen 9 vorgesehen, durch welche entsprechende Befestigungschrauben hindurchgeführt werden können.

Die Wanddicke der Schaufeln 3, 4 nimmt in radialer Richtung zum Nabenbereich 5 hin ab. Am Außenumfang des Laufrades beträgt die Wandicke vorzugsweise ca. 1,2mm. Die Wanddicke der längeren Schaufeln beträgt in ihrem nabennahen Enbereich hier ca. 0.6 mm.

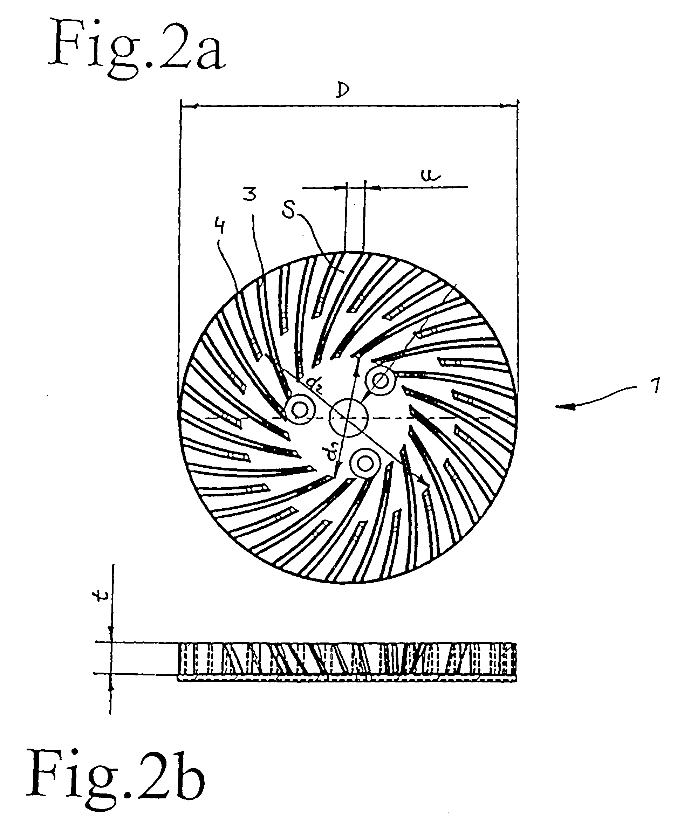

In Fig. 2a ist eine Draufsicht des Laufrades gem. Fig.1 dargestellt. Die Anzahl der Schaufeln 3, 4 ist derart abgestimmt, daß die Breite u eines Schaufelkanales S am Außenumfang des Radial-Rades weniger als 2,5% des Außenumfanges des Laufrades beträgt.

Der durch die längeren Schaufeln 3 definierte Laufradinnenkreis weist einen Durchmesser d1 auf der hier im Bereich von 25% bis 40% des Laufradaußendurchmessers D, liegt.

Der durch die kürzeren Schaufeln 4 definierte Laufradinnenkreis weise einen Durchmesser d2 auf der hier im Bereich von 50% bis 60% des Laufradaußendurchmessers D liegt. Das Laufrad 1 ist hier abgesehen von den Wanddicken der Schaufeln im 3, 4 im wesentlichen maßstäblich dargestellt. Hinsichtlich einer bevorzugten Schaufelkrümmung wird hiermit auf die bildliche Darstellung nach Fig.2a verwiesen.

In Fig.2b ist eine Seitenansicht des Laufrades nach Fig.2a dargestellt. Das Laufrad weist eine axiale Tiefe t auf die hier im Bereich von 8 bis 20 % des Laufradaußendurchmessers D liegt. Bei der hier dargestellten Ausführungsform ist die axiale Tiefe t der Schaufelkanäle in radialer Richtung konstant.

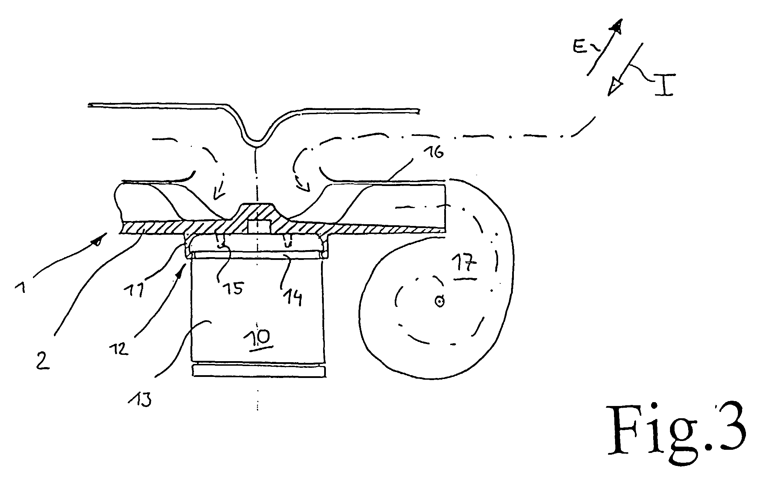

In Fig.3 ist in Form einer stark vereinfachten Skizze eine Axialschnittansicht durch ein Laufrad 1 dargestellt das hier über eine Rast/Klemmbefestigungsstruktur mit einem Motor 10 gekoppelt ist. Das Laufrad 1 weist hierzu einen dem Motor 10 zugewandten Ringabschnitt 11 auf. Der Ringabschnitt 11 ist mit einer Rastnase 12 versehen die in eine komplementär an einem Außenläufer 13 des Motores ausgebildete Umfangsnut 14 eingreift.

Weiterhin sind integral mit dem Laufrad 1 ausgebildete Mitnahmeelemente 15 vorgesehen die mit Bohrungen in Eingriff bringbar sind die in einer Stirnseite des Außenläufers ausgebildet sind. Durch diese Mitnahmeelemente 15 wird zusätzlich zu der im wesentlichen reibschlüssigen Koppelung über die Rast/Klemmbefestigungsstruktur eine Drehmomentenübertragung zwischen Laufrad 1 und Außenläufer 13 sichergestellt.

Das Laufrad 1 ist hier als einseitig offenes Radial-Laufrad ausgebildet. Die einseitige Abdeckung wird durch den bereits in Verbindung mit Fig.1 angesprochenen Basiskörper 2 erreicht. Der Basiskörper 2 weist bei der hier dargestellten Ausführungsform eine zum Außenumfang hin abnehmende Dicke auf. Hierdurch wird eine selbststabilisierende Kreiselwirkung erreicht.

Die Abdeckung der offenen axialen Stirnseite des Laufrades 1 erfolgt hier über eine stationäre Abdeckplatte 16 die über einen geringen Bewegungsspalt an das Laufrad herangeführt ist.

Die Abdeckplatte bildet hier eine sich trichterförmig zum Laufrad hin aufweitende Zustömöffnung.

Die Ableitung der durch das Laufrad geförderten Luft erfolgt vorzugsweise über eine Kanaleinrichtung 17 die bezogen auf den Umfangsbereich des Laufrades eine gleichmäßige und von Druckspitzen freie Ableitung des Atemgases erlaubt.

Der Verlauf des Strömungsweges durch die im Zusammenspiel mit dem Laufrad gebildete Gebläseeinrichtung ist hier durch Strichpunktlinien angedeutet. Der Pfeil I verdeutlicht die Strömungsrichtung während einer Inspirationsphase. Der Pfeil E kennzeichnet die Strömungsrichtung des Atemgases während einer Expirationsphase.

Das erfindungsgemäß ausgestaltete Laufrad bildet eine Druckschleuse deren Differenzdruckniveau präzise durch die Laufraddrehzahl abgestimmt werden kann. Durch die erfindungsgemäße Schaufelanordnung erhält das Laufrad eine Fördercharakteristik die bei vergleichsweise geringem Regelungsbedarf den physiologischen Bedürfnissen des Patienten verbessert Rechnung trägt. Aufgrund der durch das erfindungsgemäß ausgestaltete Laufrad erreichten hohen Laufruhe kann der Schalldämpfungsaufwand verringert werden. Hierdurch wird eine deutliche Verringerung des respiratorischen Widerstandes erreicht.

## Patentansprüche

1. Vorrichtung zur Zufuhr eines Atemgases mit einer Gebläseeinrichtung, die eine Antriebseinrichtung und ein Laufrad aufweist, wobei das Laufrad mehrere, durch Schaufeln voneinander getrennte Schaufelkanäle aufweist, **dadurch gekennzeichnet, daß** die Anzahl der Schaufeln derart festgelegt ist, daß sich am Außenumfang des Rades eine Schaufelkanalbreite im Bereich von 0,1 - 6,5 % des Außenumfangs des Laufrades ergibt.

2. Vorrichtung nach Anspruch 1, **dadurch gekennzeichnet, daß** das Laufrad ein Radiallaufrad ist.

3. Vorrichtung nach Anspruch 2, **dadurch gekennzeichnet, daß** die Schaufeln unterschiedliche radiale Schaufeltiefen aufweisen.

4. Vorrichtung nach einem der Ansprüche 1 - 3, **dadurch gekennzeichnet, daß** in Umfangsrichtung Schaufeln mit großer Schaufeltiefe und Schaufeln mit kleiner radialer Schaufeltiefe abfolgend angeordnet sind.

5. Vorrichtung nach einem der Ansprüche 1 - 4, **dadurch gekennzeichnet, daß** die radiale Schaufeltiefe der kleinen Schaufeln im Bereich von 30 - 70 % der radialen Schaufeltiefe der großen Schaufeln liegt.

6. Vorrichtung nach einem der Ansprüche 1 - 5, **dadurch gekennzeichnet, daß** die axiale Schaufeltiefe (t) der Schaufelkanäle im Bereich von 5 - 20 % des Laufraddurchmessers (D) liegt.

7. Vorrichtung nach einem der Ansprüche 1 - 6, **dadurch gekennzeichnet, daß** die axiale Schaufeltiefe (t) der Schaufelkanäle entlang des Verlaufs des Schaufelkanals in radialer Richtung im wesentlichen konstant ist.

8. Vorrichtung nach einem der Ansprüche 1 - 7, **dadurch gekennzeichnet, daß** die Schaufeltiefe (t) der Schaufelkanäle sich im Verlauf des Schaufelkanals in radialer Richtung, ändert.

9. Vorrichtung nach einem der Ansprüche 1 - 8, **dadurch gekennzeichnet, daß** die axiale Schaufeltiefe (t) der Schaufelkanäle in radialer Richtung nach außen hin abnimmt.

10. Vorrichtung nach einem der Ansprüche 1 - 9, **dadurch gekennzeichnet, daß** das Laufrad einseitig offen ausgebildet ist.

11. Vorrichtung nach einem der Ansprüche 1 - 10, **dadurch gekennzeichnet, daß** der Innendurchmesser des Zutrittsbereiches im Bereich von 25 - 66 % des Laufraddurchmessers (D) liegt.

12. Vorrichtung nach einem der Ansprüche 1 - 11, **dadurch gekennzeichnet, daß** das Laufrad aus einem Kunststoffmaterial gebildet ist.

13. Vorrichtung nach einem der Ansprüche 1 - 12, **dadurch gekennzeichnet, daß** die Wandungsfläche der die Schaufelkanäle begrenzenden Wandung eine hohe Oberflächenrauhigkeit aufweist.

14. Laufrad für eine Gebläseeinrichtng zum Aufbau eines Überdruckes in Atemgas insbesondere für ein CPAP-Gerät, wobei das Laufrad eine Vielzahl von Laufschaufeln aufweist, **dadurch gekennzeichnet, daß** die Laufschaufeln unterschiedliche radiale Schaufeltiefen aufweisen.

15. Laufrad nach Anspruch 14, **dadurch gekennzeichnet, daß** zwischen zwei Laufschaufeln großer radialer Tiefe wenigstens eine Laufschaufel verminderter radialer Tiefe angeordnet ist.

16. Laufrad nach Anspruch 14 oder 15, **dadurch gekennzeichnet, daß** ein zwischen zwei Laufschaufeln großer radialer Tiefe begrenzter Kanalbereich durch radial kürzere und/oder axial niedrigere Schaufelelemente unterteilt ist.

17. Laufrad nach wenigstens einem der Ansprüche 14 - 16, **dadurch gekennzeichnet, daß** die Wanddicke der Laufschaufeln zum Nabenbereich hin abnimmt.

18. Laufrad nach wenigstens einem der Ansprüche 14 - 17, **dadurch gekennzeichnet, daß** die Wanddicke der Laufschaufeln am Außenumfang im Bereich von 0,6 - 1,8 mm liegt.

19. Laufrad nach wenigstens einem der Ansprüche 14 - 18, **dadurch gekennzeichnet, daß** die Laufschaufeln schwach rückwärts gekrümmt ausgebildet sind.

20. Laufrad nach wenigstens einem der Ansprüche 14 bis 19, **dadurch gekennzeichnet, daß** das Laufrad im Rahmen eines Kunststoffspritzvorganges gefertigt ist.
